## Europäisches Patentamt

## European Patent Office

## Office européen des brevets

(11) Numéro de publication: **0 139 601**

**B1**

(12) **FASCICULE DE BREVET EUROPÉEN**

(45) Date de publication du fascicule du brevet:
08.04.87

(21) Numéro de dépôt: **84420166.5**

(22) Date de dépôt: **05.10.84**

(51) Int. Cl.⁴: **C 07 D 301/32**, C 07 D 303/04

(54) Procédé et dispositif pour concentrer des solutions aqueuses d'oxyde d'éthylène.

(30) Priorité: **19.10.83 FR 8316615**

(43) Date de publication de la demande:
**02.05.85 Bulletin 85/18**

(45) Mention de la délivrance du brevet:
**08.04.87 Bulletin 87/15**

(84) Etats contractants désignés:
**BE DE FR GB IT NL SE**

(56) Documents cité:
**US-A-3 745 092**

(73) Titulaire: **ATOCHEM, 4 & 8, Cours Michelet La Défense 10, F-92800 Puteaux (FR)**

(72) Inventeur: **Neel, Henry, 7 Boul. François 1er, F-76600 Le Havre (FR)**
Inventeur: **Delannoy, Francis, 61 rue Ampère, F-69310 Pierre- Bénite (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

La présente invention concerne la fabrication de l'oxyde d'éthylène. Plus précisément, l'invention concerne un procédé pour concentrer des solutions aqueuses d'oxyde d'éthylène telles que, par exemple, celles obtenues par absorption à l'eau de l'oxyde d'éthylène pour le séparer du mélange réactionnel de synthèse.

A l'échelle industrielle, l'oxyde d'éthylène est généralement préparé par oxydation en phase gazeuse de l'éthylène par l'oxygène en présence d'un catalyseur à base d'argent. L'oxyde d'éthylène étant très dilué dans le mélange gazeux issu de la réaction d'oxydation, ce mélange doit être ultérieurement soumis à divers traitements (absorptions, distillations, flash, etc...) pour obtenir l'oxyde d'éthylène pur (voir par exemple les brevets FR 1 343 492, FR 2 305 436 et US 3 904 656).

Dans la pratique, l'isolement de l'oxyde d'éthylène du mélange gazeux issu de la synthèse se fait en plusieurs étapes comprenant:

(a) une absorption à l'eau: on met en contact le mélange gazeux avec de l'eau dans une colonne comprenant plusieurs étages théoriques ou dispositifs de contact gaz-liquide. On obtient de cette façon une solution aqueuse contenant environ 2,5 % en poids d'oxyde d'éthylène, ainsi que des gaz dissous ($CO_2$, $CH_4$, $C_2H_4$, azote, argon,...) et autres impuretés (principalement formaldéhyde et acétaldéhyde).

(b) une désorption: la solution aqueuse ci-dessus est "strippée" dans une colonne de distillation, munie ou non d'une section d'enrichissement. On obtient en pied un courant aqueux ne contenant plus d'oxyde d'éthylène et en tête un melange d'oxyde d'éthylène, de vapeur d'eau et des gaz dissous et autres impuretés initialement présents dans la solution aqueuse d'oxyde d'éthylène. Ce courant gazeux a une teneur en oxyde d'éthylène d'environ 30 à 60 % en poids.

(c) une réabsorption: Le courant gazeux précédent est refroidi, puis mis en contact avec de l'eau pour réabsorber l'oxyde d'éthylène. La plus grande partie des gaz dissous n'est pas réabsorbée dans l'eau et est facilement séparée sous forme de courant gazeux. On obtient une solution aqueuse d'oxyde d'éthylène dont la concentration est comprise entre 5 et 15 % en poids.

(d) Une distillation de cette solution pour obtenir l'oxyde d'éthylène pur.

Il est évident que l'énergie à fournir pour séparer les deux constituants de cette solution est d'autant plus grande que la teneur en oxyde d'éthylène est faible. Il est donc dommage, alors que le mélange gazeux issu de l'étape ( b) contient environ 30 à 60 % en poids d'oxyde d'éthylène, de rajouter de l'eau pour le réabsorber et donc dépenser ensuite une énergie considérable pour séparer cette eau de l'oxyde d'éthylène. La présente invention a donc pour but de pallier cet inconvénient de la technique antérieure et, plus spécialement, de passer du courant gazeux contenant environ 30 à 60 % d'oxyde d'éthylène en poids à une solution aqueuse contenant au moins 95 % en poids d'oxyde d'éthylène et généralement plus de 97 %.

Conformément à la présente invention, on parvient à ce résultat en condensant progressivement le courant gazeux issu de l'étape (b) dans deux ou, de préférence, trois échangeurs de chaleur disposés en série.

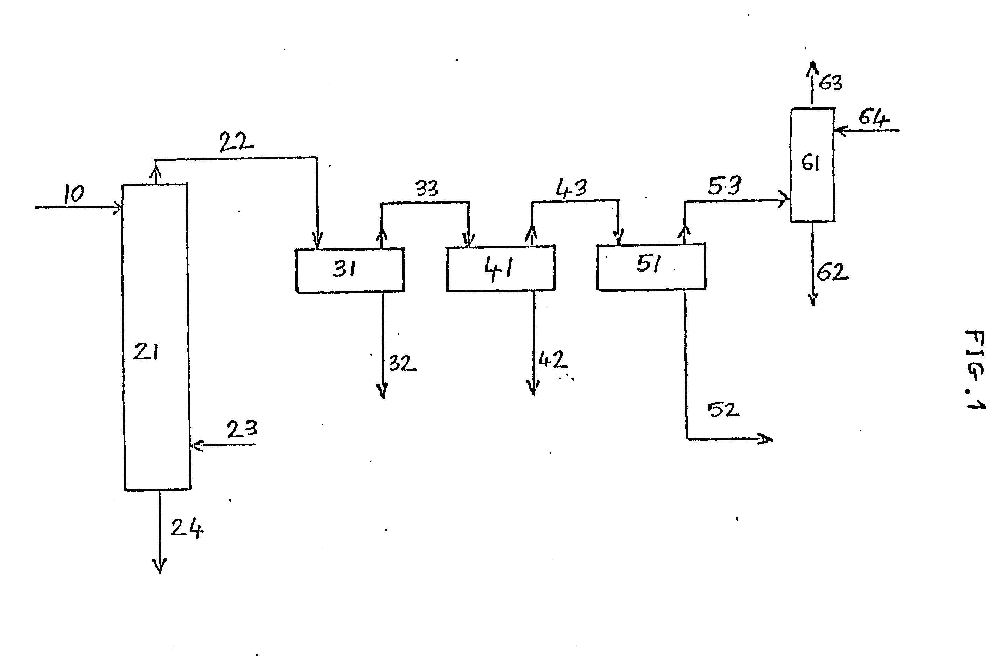

La figure 1 sur laquelle, par souci de simplification et de clarté, on n'a pas représenté les parties accessoires telles que pompes, vannes, etc..., est un schéma d'un dispositif pour la mise en oeuvre du procédé préféré selon l'invention. Ce dispositif comprend essentiellement et successivement une colonne de distillation 21, trois échangeurs 31, 41 et 51, et une colonne d'absorption 61.

La colonne 21 est une colonne de distillation conventionnelle comportant plusieurs étages théoriques ou dispositifs de contact gaz-liquide et est munie ou non d'une section d'enrichissement. Dans la partie supérieure de cette colonne 21, on introduit comme courant 10 une solution aqueuse d'oxyde d'éthylène très diluée contenant éventuellement des gaz dissous (en général moins de 0,25 % en poids) et autres impuretés. En 24, on obtient un courant aqueux débarrassé de l'oxyde d'éthylène, des gaz dissous et des impuretés. En 22, on obtient un mélange d'oxyde d'éthyléne (environ 30 à 60 % en poids) et de vapeur d'eau, ainsi que la majeure partie des gaz dissous et des impuretés éventuellement présents dans le courant 10. L'énergie de séparation est fournie par une injection de vapeur 23 en pied de la colonne 21, ou par un rebouilleur alimenté par une source chaude quelconque. La colonne 21 fonctionne de façon classique et conventionnelle.

Le courant gazeux 22 est ensuite condensé dans trois échangeurs disposés en série. Les deux premiers (31 et 41) réalisent une condensation partielle et le troisième (51) une condensation totale de l'oxyde d'éthylène. On recueille en 52 une phase liquide contenant plus de 95 % en poids d'oxyde d'éthy -lène, un peu d'eau, une petite partie du $CO_2$ dissous dans le courant 10 et quelques impuretés (principalement du formaldéhyde et de l'acétaldéhyde). En 53, on recueille une phase gazeuse formée de la majeure partie des gaz dissous et une faible partie des impuretés contenues dans 10, mélangées à des vapeurs d'oxyde et d'eau. Ce courant 53 est lavé dans une colonne d'absorption 61 possédant quelques étages théoriques ou dispositifs de contact gaz-liquide, par un courant d'eau 64 pour récupérer en 62 une solution aqueuse d'oxyde d'éthylène et en 63 une partie des gaz dissous dans 10.

La colonne 21 ainsi que ses dispositifs de contact gazliquide et les échangeurs 31, 41 et 51 sont dimensionnés selon les règles de l'art. La pression de la colonne 21 et des échangeurs 31, 41 et 51 est la même aux pertes de charges près, dues à la circulation des fluides dans les appareils 21, 31, 41 et 51 et leurs tuyauteries. La pression absolue de l'ensemble est maintenue entre 1,5 et 6 bars, avantageusement entre 1,6 et 4 bars et de préférence entre 1,9 et 3 bars. On règle la température de la source froide de l'échangeur 51 entre 5°C et une température maximale inférieure de 5°C à celle de condensation de l'oxyde d'éthylène pur à la pression considérée. Cette source froide peut être de l'air selon les conditions climatiques du lieu de fonctionnement de l'échangeur, ou plus souvent de l'eau de refroidissement.

Le courant 22 dont la température est généralement comprise entre 90 et 150°C, est condensé partiellement dans l'échangeur 31 de telle sorte que la phase liquide 32 ne contienne pas plus de 12 % de l'oxyde d'éthylène contenu dans le courant 22. On obtient facilement ce réglage en agissant sur la source froide de l'échangeur 31. La source froide peut être par exemple de l'eau de refroidissement entre 10 et 40°C.

L'échangeur 41 est utilisé comme condenseur partiel pour récupérer, dans la phase gazeuse de sortie 43, le majeure partie de l'oxyde d'éthylène introduit par le courant 33, tout en maintenant dans cette phase 43 une quantité d'eau inférieure à 5 % par rapport au poids d'oxyde d'éthylène. On règle ces proportions en agissant sur la source froide de l'échangeur 41. Pour trouver le réglage de température, on commence à condenser à une température légèrement inférieure à celle du courant 33, puis, tout en mesurant les quantités d'eau et d'oxyde d'éthylène dans les phases 42 et 43, on abaisse progressivement cette température jusqu'à l'obtention de la concentration voulue en oxyde d'éthylène dans la phase gazeuse 43. La source froide de l'échangeur 41 peut être par exemple de l'eau de refroidissement entre 10 et 40°C.

Le courant 43 est condensé dans l'échangeur 51. En 53, on recueille la majeure partie des gaz éventuellement dissous dans le courant 10 et une faible partie des impuretés du courant 10 mélangées à des vapeurs d'oxyde et d'eau. En 52, on recueille presque tout l'oxyde d'éthylène du courant 43 (sauf la faible part éventuellement entrainée dans le courant 53), de l'eau, quelques impuretés et une petite partie du $CO_2$ initialement dissous dans le courant 10.

Le courent 52 qui contient plus de 95 % en poide d'oxyde d'éthylène, et en général plus de 97 %, peut être utilisé tel quel ou, selon sa destination finale, être purifié si on le désire.

Le courant 53 est lavé dans la colonne d'absorption 61 par de l'eau (courant 64) prise entre 5 et 50°C. On peut remplacer l'eau par une solution aqueuse de glycol disponible dans le procédé. La colonne 61 fonctionne de façon conventionnelle, le débit du courant 64 étant choisi en fonction du nombre d'étages théoriques ou de dispositifs de contact gaz-liquide de la colonne. En général, le courant 53 représente moins de 5 % en poids, le plus souvent moins de 1 %, du courant 10; il suffit de trois à dix étages théoriques pour récupérer la majeure partie de l'oxyde d'éthylène du courant 53. On recueille un gaz 63 contenant la majeure partie des gaz dissous dans le courant 10 et un peu de vapeur d'eau. Après compression, ce gaz qui contient une proportion importante d'éthylène peut être renvoyé dans la zone réactionnelle. On recueille d'autre part un liquide 62 consistant en une solution aqueuse d'oxyde d'éthylène qu'on peut mélanger avec les courants 32 et 42. Ce mélange peut être traité thermiquement pour faire du glycol ou distillé pour préparer de l'oxyde d'éthylène pur ou encore remélangé au courant 10 si on ne s'intéresse qu'à la production d'oxyde d'éthylène concentré (52). Le courant 24 peut, après refroidissement, être utilisé pour absorber l'oxyde d'éthylène (étape a) et revenir dans le colonne 21 comme courent 10.

L'échengeur 31 peut éventuellement être supprimé. Dans ce cas le fonctionnement reste le même à l'exception du réglage de la source froide de l'échangeur 41. On règle cette source froide de façon à recueillir dans la phase gazeuse 43 la majeure partie de l'oxyde d'éthylène du courant 22, en veillent cependant à ce que le quantité d'eau dans la phase 43 reste inférieure à 5 % du poids d'oxyde d'éthylène. Pour régler la température de la phase 43, on opère comme indiqué précédemment, mais en se basant sur la température du courant 22 au lieu de celle du courant 33.

Les tableaux 1 à VI suivants résument les conditions opératoires et les résultats obtenus dans six exemples illustrant l'invention, sans la limiter. Les signes ≠ et ≅ ont les significations suivantes:

≅ : environ

≠ : traces

les numéros des courants matière correspondent à ceux de la figure 1. Dans les bilans on n'a pas indiqué les impuretés (telles que formaldéhyde et acétaldéhyde) qui sont à l'état de traces et suivent le même chemin que l'oxyde d'éthylène.

Les exemples 1, 2 et 3 correspondant respectivement aux tableaux I, II et III ont été réalisés dans les mêmes conditions de pression (2,7 à 2,5 bars) à partir de solutions aqueuses d'oxyde d'éthylène contenant des quantités décroissantes de gaz dissous.

L'exemple 4 (tableau IV) a été réalisé à partir de la même solution aqueuse d'oxyde d'éthylène qu'à l'exemple 1, mais en opérant à une pression plus élevée (3,45 à 3,25 bars).

L'exemple 5 reproduit l'exemple 3, mais en operant a une pression beaucoup plus faible (2 à 1,8 bars). L'examen des résultats indiqués sur le tableau V montre, en comparaison avec ceux de l'exemple 4, qu'on obtient des débits 52 et 53 semblables bien qu'en travaillant à une pression presque moitié moins élevée.

L'exemple 6 (tableau VI) illustre l'emploi de seulement deux échangeurs (41 et 51).

**Tableau I**

| COURANT N°- | 10 | 22 | 32 | 33 | 42 | 43 | 52 | 53 |
|---|---|---|---|---|---|---|---|---|
| Pression absolue $(kg/cm^2)$ | 2,7 | 2,7 | 2,6 | 2,6 | 2,54 | 2,54 | 2,5 | 2,5 |
| Température (°C) | 108 | 114 | 75 | 75 | 42 | 42 | 25 | 25 |
| Débit (kg/h) | 1900 | 88,54 | 36,54 | 52 | 4,36 | 47,6 | 34,2 | 13,4 |
| Composition (% en pds) | | | | | | | | |
| Oxyde d'éthylène | ≈ 2,6 | 54,67 | 11,14 | 85,26 | 35,29 | 89,84 | 97,47 | 70,3 |
| Eau | ≈ 92 | 40,43 | 88,71 | 6,51 | 64,7 | 1,19 | 1,57 | 0,21 |
| Ethylène Glycol | 5,2 | 0,05 | 0,13 | * | * | * | * | * |
| $CO_2$ | 0,17 | 3,73 | * | 6,34 | 0,013 | 6,9 | 0,79 | 22,6 |
| $C_2H_4$ | 0,032 | 0,69 | * | 1,18 | * | 1,3 | 0,16 | 4,2 |
| $N_2$, $CH_4$, Argon ... | 0,019 | 0,43 | * | 0,71 | * | ≈ 0,77 | * | 2,67 |

0 139 601

Tableau II

| COURANT N°– | 10 | 22 | 32 | 33 | 42 | 43 | 52 | 53 |
|---|---|---|---|---|---|---|---|---|
| Pression absolue kg/cm² | 2,7 | 2,7 | 2,6 | 2,6 | 2,54 | 2,54 | 2,5 | 2,5 |
| Température (°C) | 108 | 115 | 75 | 75 | 42 | 42 | 25 | 25 |
| Débit (kg/h) | 1900 | 83,64 | 35 | 48,65 | 4,32 | 44,32 | 41,5 | 2,8 |
| Composition (% en poids) | | | | | | | | |
| Oxyde d'éthylène | 2,6 | 57,88 | 12,04 | 90,84 | 39,52 | 95,85 | 97,7 | 68,79 |
| Eau | 91,9 | 40,47 | 87,8 | 6,42 | 60,46 | 1,15 | 1,22 | 0,17 |
| Ethylène glycol | 5,43 | 0,06 | 0,13 | ✗ | ✗ | ✗ | ✗ | ✗ |
| $CO_2$ | 0,065 | 1,47 | ✗ | 2,53 | · | 2,77 | 1,01 | 28,43 |
| $C_2H_4$ | 0,003 | 0,067 | ✗ | 0,115 | ✗ | 0,12 | 0,05 | 1,26 |
| $N_2$, $CH_4$, Argon | 0,002 | 0,048 | ✗ | 0,08 | ✗ | 0,09 | ✗ | 1,34 |

**Tableau III**

| COURANT N°- | 10 | 22 | ·32 | 33 | 42 | 43 | 52 | 53 |
|---|---|---|---|---|---|---|---|---|
| Pression absolue $(kg/cm^2)$ | 2,7 | 2,7 | 2,6 | 2,6 | 2,54 | 2,54 | 2,5 | |
| Température (°C) | 108 . | 115 | 75 | 75 | 42 | 42 | 25 | |
| Débit (kg/h) | 1900 | 78,2 | 33,9 | 44,3 | 4,04 | 40,25 | 40,3 | 0 |
| Composition (% en poids) | | | | | | | | |
| Oxyde d'éthylène | 2,5 | 57,97 | 12,31 | 92,93 | 40,9 | 98,15 | ·98,15 | |
| Eau | 92,25 | 41,61 | 87,55 | 6,43 | 59,1 | 1,15 | 1,15 | |
| Éthylène glycol | 5,24 | 0,05 | · 0,13 | x | x | x | | |
| $CO_2$ | 0,0016 | 0,039 | x | 0,07 | x | 0,076 | 0,076 | |
| $C_2H_4$ | 0,013 | 0,32 | x | 0,56 | x | 0,62 | 0,62 | |
| $N_2$, $CH_4$, Argon | x | x | x | x | x | x | x | |

0 139 601

**Tableau IV**

| COURANT N°– | 10 | 22 | 32 | 33 | 42 | 43 | 52 | 53 |
|---|---|---|---|---|---|---|---|---|
| Pression absolue $(kg/cm^2)$ | 3,45 | 3,45 | 3,35 | 3,35 | 3,29 | 3,29 | 3,25 | 3,25 |
| Température (° C) | 108 | 119 | 75 | 75 | 42 | 42 | 25 | 25 |
| Débit (kg/h) | 1900 | 79,74 | 29 | 50,72 | 5,33 | 45,4 | 37,36 | 8,04 |
| Composition (% en poids) | | | | | | | | |
| Oxyde d'éthylène | 2,6 | 60,71 | 15,26 | 86,7 | 60,83 | 89,75 | 97,2 | 55,16 |
| Eau | 92 | 33,87 | 84,6 | 4,86 | 39,15 | 0,83 | 0,99 | 0,11 |
| Èthylène glycol | 5,2 | 0,05 | 0,133 | x | x | x | x | x |
| $CO_2$ | 0,17 | 4,13 | x | 6,5 | 0,015 | 7,26 | 1,5 | 34,01 |
| $C_2H_4$ | 0,032 | 0,77 | x | 1,21 | x | 1,35 | 0,296 | 6,27 |
| $N_2$, $CH_4$, Argon | 0,019 | 0,45 | x | 0,71 | x | 0,8 | x | 4,42 |

0 139 601

**Tableau V**

| COURANT N°- | 10 | 22 | 32 | 33 | 42 | 43 | 52 | 53 |
|---|---|---|---|---|---|---|---|---|
| Pression absolue $(kg/cm^2)$ | 2 | 2 | 1,9 | 1,9 | 1,84 | 1,84 | 1,8 | 1,8 |
| Température (°C) | 108 | 110 | 75 | 75 | 42 | 42 | 25 | 25 |
| Débit (kg/h) | 1900 | 97,49 | 51,9 | 45,58 | 4,76 | 40,81 | 33,78 | 7,04 |
| Composition (% en poids) | | | | | | | | |
| Oxyde d'éthylène | 2,5 | 46,5 | 8,23 | 90,1 | 25,72 | 97,6 | 97,9 | 96,15 |
| Eau | 92,25 | 53,14 | 91,64 | 9,3 | 74,27 | 1,71 | 1,99 | 0,35 |
| Ethylène glycol | 5,24 | 0,069 | 0,129 | X | X | X | X | X |
| $CO_2$ | 0,0016 | 0,031 | X | 0,067 | X | 0,075 | 0,01 | 0,39 |
| $C_2H_4$ | 0,013 | 0,25 | X | 0,54 | X | 0,61 | 0,091 | 3,11 |
| $N_2$, $CH_4$, Argon | X | X | X | X | X | X | X | X |

**Tableau VI**

| COURANT N°- | 10 | 22 | 32 | 33 | 42 | 43 | 52 | 53 |
|---|---|---|---|---|---|---|---|---|
| Pression absolue (kg/cm2) | 2,7 | 2,7 | | | 2,54 | 2,54 | 2,5 | 2,5 |
| Température (°C) | 108 | 115 | | | 42 | 42 | 25 | 25 |
| Débit (kg/h) | 1900 | 83,64 | | | 54,57 | 29,07 | 25,71 | 3,37 |
| Composition (% en poids) | | | | | | | | |
| Oxyde d'éthylène | 2,6 | 57,88 | | | 38,5 | 94,28 | 97,63 | 68,71 |
| Eau | 91,9 | 40,47 | | | 61,41 | 1,16 | 1,28 | 0,17 |
| Éthylene glycol | 5,43 | 0,057 | | | 0,087 | X | X | X |
| $CO_2$ | 0,064 | 1,47 | | | X | 4,22 | 1,02 | 28,64 |
| $C_2H_4$ | 0,003 | 0,067 | | | X | 0,193 | 0,05 | 1,28 |
| $N_2$, $CH_4$, Argon | 0,002 | 0,049 | | | X | 0,14 | X | 1,2 |

0 139 601

## Revendications

1. Procédé de concentration d'une solution aqueuse d'oxyde d'éthylène contenant éventuellenent des gaz dissous et autres impuretés, dans lequel on soumet d'abord cette solution à un entraînement à la vapeur d'eau dans une colonne de distillation et récupère ensuite l'oxyde d'éthylène contenu dans le courant gazeux sortant en tête de ladite colonne, caractérisé en ce que l'on condense progressivement le dit courant gazeux dans deux ou trois échangeurs de chaleur disposés en série, la pression absolue de la colonne de distillation et des échangeurs étant comprise entre 1,5 et 6 bars et la température de la source froide du dernier échangeur étant comprise entre 5° C et une température maximale inférieure de 5° C à celle de condensation de l'oxyde d'éthylène pur à la pression considérée.

2. Procédé selon la revendication 1 dans lequel on utilise trois échangeurs de chaleur, caractérisé en ce que l'on règle la température de la source froide des premier et second échangeurs de telle sorte que la phase liquide sortant du premier échangeur ne contienne pas plus de 12 % de l'oxyde d'éthylène contenu dans le courant gazeux entrant dans cet échangeur et que la proportion d'eau contenue dans le courant gazeux sortant du deuxième échangeur soit inférieure à 5 % par rapport au poids d'oxyde d'éthylène.

3. Procédé selon la revendication 1 ou 2, caractérisé en ce que le courant gazeux sortant éventuellement du dernier échangeur est réabsorbé par de l'eau ou une solution aqueuse de glycol.

4. Procédé selon l'une des revendications 1 à 3, caractérisé en ce que les phases liquides fournies par le ou les échangeurs intermédiaires et éventuellement par la colonne d'absorption suivant le dernier échangeur sont remélangées avec la solution aqueuse d'oxyde d'éthylène entrant dans la colonne de distillation.

5. Procédé selon la revendication 3 ou 4, caractérisé en ce que la phase gazeuse sortant de la colonne d'absorption suivant le dernier échangeur est renvoyée dans le réacteur de synthèse d'oxyde d'éthylène.

6. Procédé selon l'une des revendications 1 à 5, caractérisé en ce que la pression absolue de la colonne de distillation et des échangeurs en série est comprise entre 1,6 et 4 bars.

7. Procédé selon la revendication 6 dans lequel la pression absolue est comprise entre 1,9 et 3 bars.

8. Dispositif pour la réalisation du procédé selon l'une des revendications 1 à 7, caractérisé en ce qu'il comprend une colonne de distillation, deux ou trois échangeurs de chaleur disposés en série et, éventuellement, une colonne d'absorption, ces appareils étant reliés entre eux par des moyens connus de telle sorte que le courant gazeux sortant en tête de la colonne de distillation circule successivement dans les échangeurs et que le courant gazeux sortant éventuellement du dernier échangeur soit introduit en bas de la colonne d'absorption.

## Patentansprüche

1. Verfahren zum Konzentrieren einer wässrigen Äthylenoxidlösung, die gegebenenfalls gelöste Gase oder andere Verunreinigungen enthält, bei dem man diese Lösung zuerst einer Wasserdampfdestillation in einer Destillationskolonne unterwirft und anschließend das Äthylenoxid zurückgewinnt, das in dem am Kopf der Kolonne austretenden Gasstrom enthalten ist, dadurch gekennzeichnet, daß man den Gasstrom nach und nach in zwei oder drei in Reihe angeordneten Wärmeaustauschern kondensiert, wobei der absolute Druck der Destillationskolonne und der Austauscher zwischen 1,5 und 6 bar und die Temperatur des Kühlwassers des letzten Austauschers zwischen 5° C und einer Maximaltemperatur von 5° C unter der Kondensationstemperatur von reinem Äthylenoxid beim gewählten Druck liegt.

2. Verfahren nach Anspruch 1, bei dem man drei Wärmeaustauscher benutzt, dadurch gekennzeichnet, daß man die Kühlwassertemperatur des ersten und des zweiten Austauschers so reguliert, daß die den ersten Austauscher verlassende flüssige Phase nicht mehr als 12 % des Äthylenoxids enthält, das in dem in diesen Austauscher eintretenden Gasstrom enthalten ist, und daß die im austretenden Gasstrom des zweiten Austauschers enthaltene Wassermenge weniger als 5 % des Äthylenoxidgewichts beträgt.

3. Verfahren nach Anspruch 1 oder 2, dadurch gekennzeichnet, daß der gegebenenfalls den letzten Austauscher verlassende Gasstrom durch Wasser oder eine wässrige Glycoilösung wieder absorbiert wird.

4. Verfahren nach einem der Ansprüche 1 bis 3, dadurch gekennzeichnet, daß die flüssigen Phasen, die in dem oder den Zwischenaustauschern und eventuell in der dem letzten Austauscher folgenden Absorptionskolonne anfallen, wieder mit der in die Destillationskolonne eintretenden wässrigen Äthylenoxidlösung gemischt werden.

5. Verfahren nach Anspruch 3 oder 4, dadurch gekennzeichnet, daß die Gasphase, die aus der dem letzten Austauscher folgenden Absorptionskolonne austritt, in den Reaktor der Äthylenoxid-Synthese zurückgeleitet wird.

6. Verfahren nach einem der Ansprüche 1 bis 5, dadurch gekennzeichnet, daß der absolute Druck der Destillationskolonne und der in Reihe angeordneten Austauscher zwischen 1,6 und 4 bar beträgt.

7. Verfahren nach Anspruch 6, in dem der absolute Druck zwischen 1,9 und 3 bar liegt.

8. Vorrichtung zur Durchführung des Verfahrens nach einem der Ansprüche 1 bis 7, dadurch gekennzeichnet, daß sie eine Destillationskolonne, zwei oder drei in Reihe angeordnete Wärmeaustauscher und eventuell eine Absorptionskolonne enthält, wobei diese Apparate durch bekannte Mittel so miteinander verbunden sind, daß der am Kopf der Destillationskolonne austretende Gasstrom nacheinander durch die Austauscher strömt, und daß der gegebenenfalls aus dem letzten Austauscher austretende Gasstrom am Fuß der Absorptionskolonne eingeleitet wird.

**Claims**

1. A process for the concentration of an aqueous solution of ethylene oxide which may contain dissolved gases and other impurities, wherein said solution is first subjected to entrainment with steam in a distillation column, the recovery being then effected of the ethylene oxide contained in the gas stream issuing from the head of said column, characterised in that said gas flow is progressively condensed in two or three heat exchangers arranged in series, the absolute pressure of the distillation column and of the exchangers being comprised between 1.5 and 6 bar and the temperature of the cold source of the last exchanger being comprised between 5°C and a maximum temperature which is 5°C below the temperature of condensation of pure ethylene oxide at the pressure under consideration.

2. A process according to Claim 1 wherein use is made of three heat exchangers, characterised in that the temperature of the cold source of the first and second exchangers is regulated in such a manner that the liquid phase issuing from the first exchanger does not contain more than 12% of the ethylene oxide contained in the gas stream entering said exchanger and that the proportion of water contained in the gas stream issuing from the second exchanger is less than 5% relative to the weight of ethylene oxide.

3. A process according to Claims 1 or 2, characterised in that the gas stream eventually issuing from the last exchanger is reabsorbed by water or an aqueous glycol solution.

4. A process according to one of Claims 1 to 3, characterised in that the liquid phases supplied by the intermediate exchanger(s) and eventually by the absorption column following the last exchanger are again mixed with the aqueous solution of ethylene oxide entering the distillation column.

5. A process according to Claims 3 or 4, characterised in that the gas phase issuing from the absorption columm following the last exchanger is sent back into the ethylene oxide synthesis reactor.

6. A process according to one of Claims 1 to 5, characterised in that the absolute pressure of the distillation column and of the series-mounted exchangers is comprised between 1.6 and 4 bar.

7. A process according to Claim 6 wherein the absolute pressure is comprised between 1.9 and 3 bar.

8. A device for carrying out the process according to one of Claims 1 to 7, characterised in that it comprises a distillation column, two or three heat exchangers arranged in series and, optionally, an absorption column, these appliances being joined together by known means in such a manner that the gas stream issuing at the head of the distillation column circulates successively in the exchangers, and the gas stream eventually issuing from the last exchanger is introduced at the bottom of the absorption column.

FIG.1